# FASCICULE DE BREVET EUROPEEN

(11) **EP 3 019 497 B1**
(45) Date de publication et mention de la délivrance du brevet: **12.04.2017**
(21) Numéro de dépôt: 14758600.2
(22) Date de dépôt: 11.07.2014
(51) Int. Cl.: C07D 417/14, A61K 31/5377, A61K 31/4025, A61K 31/403, A61P 35/00

(54) **NOUVEAU SEL DE LA 3-[(3-{[4-(4-MORPHOLINYLMÉTHYL)-1H-PYRROL-2-YL]MÉTHYLÈNE}-2-OXO-2,3-DIHYDRO-1H-INDOL-5-YL)MÉTHYL]-1,3-THIAZOLIDINE-2,4-DIONE, SA PRÉPARATION, ET LES FORMULATIONS QUI LE CONTIENNENT**
NEUARTIGES SALZ AUS 3-[(3-{[4-(4-MORPHOLINYLMETHYL)-1H-PYRROL-2-YL]METHYLEN}-2-OXO-2,3-DIHYDRO-1H-INDOL-5-YL)METHYL]-1,3-THIAZOLIDIN-2,4-DION, HERSTELLUNG DAVON UND FORMULIERUNGEN DAMIT
NOVEL SALT OF 3-[(3-{[4-(4-MORPHOLINYLMETHYL)-1H-PYRROL-2-YL]METHYLENE}-2-OXO-2,3-DIHYDRO-1H-INDOL-5-YL)METHYL]-1,3-THIAZOLIDINE-2,4-DIONE, PREPARATION THEREOF AND FORMULATIONS CONTAINING SAME

(30) Priorité: 12.07.2013 FR 1356870
(43) Date de publication de la demande: 18.05.2016
(73) Titulaire: Les Laboratoires Servier, 92284 Suresnes Cedex (FR)
(72) Inventeur: LE FLOHIC, Alexandre, F-76640 Fauville en Caux (FR); GUIDOTTI, Jérôme, F-76760 Criquetot sur Ouville (FR); LETELLIER, Philippe, F-45000 Orléans (FR)
(86) Numéro de dépôt international: PCT/FR2014/051783
(87) Numéro de publication internationale: WO 2015/004395

(56) Documents cités:
- EP-A1- 2 281 822

## Description

La présente invention concerne un nouveau sel de la 3-[(3-{[4-(4-morpholinylméthyl)-1*H-*pyrrol-2-yl]méthylène}-2-oxo-2,3-dihydro-1*H*-indol-5-yl)méthyl]-1,3-thiazolidine-2,4-dione de formule (I) : 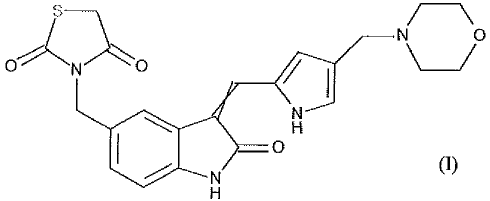 son procédé de préparation ainsi que les formulations pharmaceutiques qui le contiennent.

La 3-[(3-{[4-(4-morpholinylméthyl)-1*H*-pyrrol-2-yl]méthylène}-2-oxo-2,3-dihydro-1*H-*indol-5-yl)méthyl]-1,3-thiazolidine-2,4-dione possède des propriétés pharmacologiques très intéressantes dans le domaine de la cancérologie. En effet il a été montré que la 3-[(3-{[4-(4-morpholinylméthyl)-1*H-*pyrrol-2-yl]méthylène}-2-oxo-2,3-dihydro-1*H-*indol-5-yl)méthyl]-1,3-thiazolidine-2,4-dione possède la capacité d'inhiber la migration des cellules cancéreuses ce qui la rend particulièrement utile pour le traitement des cancers et notamment des tumeurs solides métastatiques. Parmi les traitements des cancers envisagés on peut citer, sans s'y limiter, les cancers du colon, du sein, du foie, des reins, du cerveau, de l'oesophage, les mélanomes, les myélomes, les cancers de l'ovaire, les cancers du poumon non à petites cellules, les cancers du poumon à petites cellules, les cancers de la prostate et du pancréas, les sarcomes.

La préparation et l'utilisation en thérapeutique de la 3-[(3-{[4-(4-morpholinylméthyl)-1*H-*pyrrol-2-yl]méthylène}-2-oxo-2,3-dihydro-1*H-*indol-5-yl)méthyl]-1,3-thiazolidine-2,4-dione et de ses sels d'addition à un acide pharmaceutiquement acceptable, et plus particulièrement de son chlorhydrate, ont été décrits par exemple dans le brevet européen EP 2281822.

Compte tenu de l'intérêt pharmaceutique de ce composé, il est important de pouvoir disposer du principe actif avec d'excellents rendements, une grande pureté et une excellente reproductibilité. Il s'est rapidement avéré que le chlorhydrate qui était utilisé posait des problèmes de purification et de recristallisation, ainsi qu'un rendement très difficile à optimiser. Par ailleurs, il a été noté des problèmes de reproductibilité et de constance du principe actif obtenu. Après de nombreux travaux de recherche, il a été possible de mettre en évidence un nouveau sel alliant divers avantages, notamment en matière de purification, de reproductibilité de son procédé d'obtention et de rendement, mais présentant également de façon inattendue l'avantage d'augmenter très significativement la solubilité du principe actif. Ce nouveau sel présente ainsi toutes les qualités indispensables à son utilisation en tant que médicament, tant sur le plan physicochimique que pharmacocinétique.

La présente invention concerne donc un nouveau sel de la 3-[(3-{[4-(4-morpholinylméthyl)-1*H*-pyrrol-2-yl]méthylène}-2-oxo-2,3-dihydro-1*H*-indol-5-yl)méthyl] -1,3-thiazolidine-2,4-dione et plus particulièrement le 3-[(3-{[4-(4-morpholinylméthyl)-1*H-*pyrrol-2-yl]méthylène}-2-oxo-2,3-dihydro-1*H*-indol-5-yl)méthyl]-1,3-thiazolidine-2,4-dione méthanesulfonate de formule (II) : 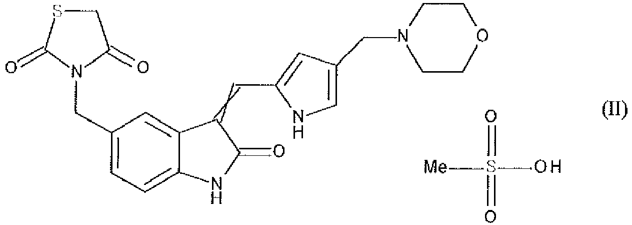 dans laquelle la notation 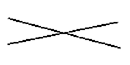 signifie que la double liaison est de configuration Z ou E.

Préférentiellement, l'invention concerne l'isomère Z du 3-[(3-{[4-(4-morpholinylméthyl)-1H-pyrrol-2-yl]méthylène}-2-oxo-2,3-dihydro-1H-indol-5-yl)méthyl]-1,3-thiazolidine-2,4-dione méthanesulfonate.

Ce nouveau sel présente les avantages suivants :
- un procédé d'obtention simple, reproductible, et avec un excellent rendement;
- une solubilité augmentée tant dans l'eau que dans les solvants organiques permettant d'envisager des étapes de purifications telles que des clarifications, afin d'en augmenter la pureté.

L'invention concerne également le procédé d'obtention du 3-[(3-{[4-(4-morpholinylméthyl)-1*H-*pyrrol-2-yl]méthylène}-2-oxo-2,3-dihydro-1*H*-indol-5-yl)méthyl] -1,3-thiazolidine-2,4-dione méthanesulfonate et plus particulièrement de son isomère Z, caractérisé en ce que l'on utilise comme produit de départ la 3-[(3-{[4-(4-morpholinylméthyl)-1*H-*pyrrol-2-yl]méthylène}-2-oxo-2,3-dihydro-1*H-*indol-5-yl)méthyl] -1,3-thiazolidine-2,4-dione, obtenue par exemple selon un procédé décrit dans le brevet EP 2281822. La dione est placée en solution dans un système binaire solvant/eau, puis 1 à 2 équivalents molaires d'acide méthane sulfonique sont ajoutés et le milieu agité jusqu'à précipitation du méthanesulfonate.
Le solvant sera avantageusement un solvant polaire comme par exemple l'acétonitrile, l'acétone, le 1,4-dioxane, le tétrahydrofurane, la N,N-diméthylformamide, la N,N-diméthylacétamide, le diméthylsulfoxide, les alcools tels que le méthanol, l'éthanol, l'isopropanol, l'eau ainsi les mélanges hydro-organiques de ces solvants. Préférentiellement le ratio solvant/eau sera de 0/100 à 100/0.

Une variante du procédé selon l'invention consiste à utiliser comme produit de départ le 3-[(3-{[4-(4-morpholinylméthyl)-1*H-*pyrrol-2-yl]méthylène}-2-oxo-2,3-dihydro-1*H*-indol-5-yl)méthyl]-1,3-thiazolidine-2,4-dione chlorhydrate, dont l'obtention a été décrite par exemple dans le brevet EP 2281822. Le chlorhydrate est mis en solution dans un système binaire solvant/eau, et le pH du milieu est ramené à 8 par addition d'une base. Le sel formé est éliminé par filtration. Le filtrat est chauffé puis l'acide méthane sulfonique est ajouté. La température est ensuite lentement ramenée à l'ambiante, et le méthanesulfonate obtenu est filtré. Plus particulièrement le solvant utilisé est un solvant polaire comme l'acétonitrile, l'acétone, le 1,4-dioxane, le tétrahydrofurane, la N,N-diméthylformamide, la N,N-diméthylacétamide, le diméthylsulfoxide, les alcools tels que le méthanol, l'éthanol, l'isopropanol. Préférentiellement le ratio solvant/eau sera de 70/30 et plus particulièrement 90/10. L'acide méthane sulfonique est utilisé en excès et plus préférentiellement 1 à 2 équivalents.

Le composé de formule (II) selon l'invention présente une excellente stabilité dans le temps même en conditions dénaturantes : à 25°C/60% d'humidité relative, à 25°C/90% d'humidité relative, à 30°C/65% d'humidité relative, à 40°C/75% d'humidité relative, ou à 50°C, le composé de formule (II) est inchangé au bout de 6 mois.

L'invention s'étend aussi aux compositions pharmaceutiques renfermant comme principe actif le composé de formule (II) selon l'invention et plus particulièrement son isomère Z, avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

Les formes pharmaceutiques contenant le composé de formule (II) selon l'invention et plus particulièrement son isomère Z, seront utilisées pour le traitement des cancers et notamment des tumeurs solides métastatiques. Parmi les traitements des cancers envisagés on peut citer, sans s'y limiter, les cancers du colon, du sein, du foie, des reins, du cerveau, de l'oesophage, les mélanomes, les myélomes, les cancers de l'ovaire, les cancers du poumon non à petites cellules, les cancers du poumon à petites cellules, les cancers de la prostate et du pancréas, les sarcomes.

La posologie utile est adaptable selon la nature et la sévérité de l'affection, la voie d'administration ainsi que l'âge et le poids du patient. Cette posologie varie de 1 mg à 1 g par jour en équivalent base en une ou plusieurs prises.

Les exemples ci-dessous illustrent l'invention, mais ne la limitent en aucune façon.

### Exemple 1 : 3-[(3-{[4-(4-Morpholinylméthyl)-1H-pyrrol-2-yl]méthylène}-2-oxo-2,3-dihydro-1H-indol-5-yl)méthyl]-1,3-thiazolidine-2,4-dione méthanesulfonate, isomère Z

1,26 g de 3-[(3-{[4-(4-morpholinylméthyl)-1*H*-pyrrol-2-yl]méthylène}-2-oxo-2,3-dihydro-1*H*-indol-5-yl)méthyl]-1,3-thiazolidine-2,4-dione sont introduits dans un ballon de 100 mL. Après addition de 20 mL d'une solution acétonitrile/eau (90/10), le mélange est chauffé à 70°C. Une solution contenant 2 mL d'acide méthanesulfonique et 50 mL d'un mélange acétonitrile/eau (90/10) est préparée. 5 mL de cette solution sont ajoutés au milieu réactionnel qui devient limpide. La solution est refroidie à 20°C (0,5°C/min, agitation 200rt/min). Après une nuit d'agitation à température ambiante, le produit du titre est isolé par filtration, séché à 40°C sous vide (10 mbars).

### Point de fusion : 270-274°C (fusion/dégradation)

Le produit du titre est caractérisé par son diagramme de poudre effectué sur 50 mg du composé de l'Exemple 1, placés entre 2 films Kapton® ou sur un support, et chargés dans un diffractomètre Panalytical Xpert-Pro MPD (anticathode de cuivre) en mode transmission avec un domaine angulaire 3-55° en 2θ, un pas de 0,017° et 35,5 s par pas, qui permet d'identifier les paramètres cristallins suivants:
- paramètres de maille : a = 15.0958(5) Å, b = 18.4586(6) Å, c = 8.8269(2) Å, β = 94.074(1)°, γ = 90°
- groupe d'espace : C 1 c 1 (9)
- volume de la maille : Vₘₐᵢₗₗₑ = 2453.37600 Å³

Le produit du titre a également été caractérisé par diffraction X sur un monocristal du composé de l'Exemple 1, effectué sur un appareil Rigaku XtaLAB utilisant des radiations Mo-Ka monochromatiques du graphite. Les paramètres cristallins suivants ont été observés :
- paramètres de maille : a = 14.995(4) Å, b = 18.302(4) Å, c = 8.850(2) Å, β = 93.528(7)°, γ = 90°
- groupe d'espace : C 1 c 1 (9)
- volume de la maille : Vₘₐᵢₗₗₑ = 2424.0 (9) Å³

Les légères différences observées avec les paramètres obtenus sur poudre proviennent de la température utilisée pour collecter les paramètres avec le monocristal (-100°C), qui induit une contraction le long des axes a et b.

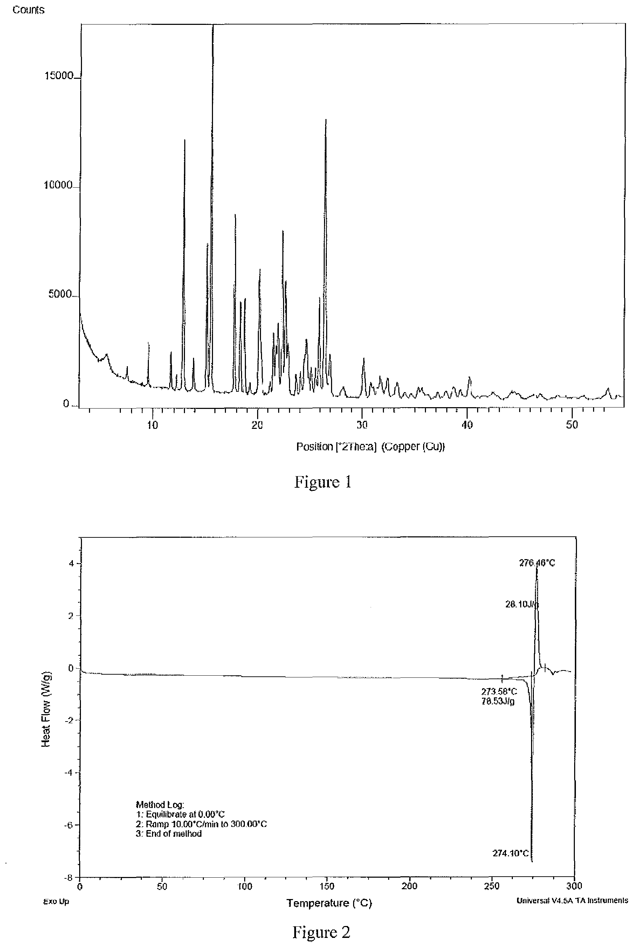

Le produit du titre a également été caractérisé par son diagramme de diffraction X sur poudre représenté dans la Figure 1, et mesuré sur un diffractomètre Panalytical Xpert Pro MPD (anticathode de cuivre) et exprimé en termes de distance inter-réticulaire d, d'angle de Bragg 2 thêta (exprimés en °±0,2), et d'intensité relative (exprimée en pourcentage par rapport à la raie la plus intense) :

| **2**-**Theta (°) exp.** | **d (Å) exp**. | **Intensité (%)** |
|---|---|---|
| 12,8678 | 6,87420 | 60,31 |
| 15,1323 | 5,85020 | 38,36 |
| 15,5005 | 5,71203 | 100,00 |
| 17,7050 | 5,00549 | 48,23 |
| 18,2579 | 4,85513 | 23,89 |
| 18,7110 | 4,73856 | 25,22 |
| 20,1109 | 4,41177 | 30,15 |
| 21,4617 | 4,13704 | 16,97 |
| 21,6776 | 4,09632 | 15,77 |
| 21,8970 | 4,05576 | 15,98 |
| 22,2971 | 3,98390 | 41,52 |
| 22,5852 | 3,93372 | 38,20 |
| 24,5702 | 3,62023 | 17,23 |
| 25,8231 | 3,44735 | 24,17 |
| 26,3301 | 3,38211 | 83,15 |

Angles de Bragg 2 thêta (exprimés en °+0,2) caractéristiques du diagramme de diffraction X sur poudre : 12,86; 15,13 ; 15,50 ; 17,70; 18,25 ; 18,71 ; 20,11 ; 21,46 ; 21,67 ; 21,89 ; 22,29 ; 22,58 ; 24,57 ; 25,82 ; 26,33.

Le composé de l'Exemple 1 a également été caractérisé par son diagramme DSC, sur un échantillon de 5-10 mg chargé dans un appareil TA instruments DSC Q1000 et refroidi à 0°C. L'échantillon est ensuite chauffé jusqu'à 300°C à la vitesse de 10°C/min. Le diagramme obtenu est représenté dans la Figure 2.

### Exemple 2 : Pureté et stabilité en conditions dénaturantes du 3-[(3-{[4-{4-morpholinyl méthyl)-1H-pyrrol-2-yl]méthylène}-2-oxo-2,3-dihydro-1H-indol-5-yl)méthyl]-1,3-thiazolidine-2,4-dione méthanesulfonate, isomère Z

| | **HPLC (% Exemple 1)** | **RX** | **DSC** |
|---|---|---|---|
| t = 0 | 99.8% | Fig. 2 | Fig.1 |
| | **Après 6 mois** | | |
| 25°C/60% humidité relative | 99.8% | Difractogramme inchangé | Thermogramme inchangé |
| 25°C/90% humidité relative | 99.8% | Difractogramme inchangé | Thermogramme inchangé |
| 30°C/65% humidité relative | 99.8% | Difractogramme inchangé | Thermogramme inchangé |
| 40°C/75% humidité relative | 99.8% | Difractogramme inchangé | Thermogramme inchangé |
| 50°C | 99.8% | Difractogramme inchangé | Thermogramme inchangé |

### Exemple 3 : Solubilité du 3-[(3-{[4-(4-morpholinylméthyl)-1H-pyrrol-2-yl]méthylène}-2-oxo-2,3-dihydro-1H-indol-5-yl)méthyl]-1,3-thiazolidine-2,4-dione méthanesulfonate, isomère Z

Une solution contenant 140 mg du composé obtenu dans l'Exemple 1 dans 7 ml d'eau est agitée pendant 24 heures à température ambiante. Après filtration sur un acrodiscs 0.45µm GHP, la solution est analysée par HPLC. La solubilité du composé de l'Exemple 1 est de 14,7 mg /ml (ou 12,1 mg /ml en équivalent base).
Dans les mêmes conditions, la solubilité du chlorhydrate de la 3-[(3-{[4-(4-morpholinylméthyl)-1*H*-pyrrol-2-yl]méthylène}-2-oxo-2,3-dihydro-1*H*-indol-5-yl)méthyl] -1,3-thiazolidine-2,4-dione, isomère Z, est de 4,3 mg /ml (ou 4 mg / ml en équivalent base).

### Exemple 4 : Cinétique de dissolution à pH 2 (pH gastrique) du 3-[(3-{[4-(4-morpholinylméthyl)-1H-pyrrol-2-yl]méthylène}-2-oxo-2,3-dihydro-1H-indol-5-yl)méthyl]-1,3-thiazolidine-2,4-dione méthanesulfonate, isomère Z

La cinétique de dissolution à surface constante (ou cinétique de dissolution intrinsèque) du produit de l'Exemple 1 a été déterminée à température ambiante à pH 2 (10 mL d'HCl 0,01N) à l'aide d'un appareil de dissolution µDiss sur des pastilles de 0,075 cm² préparées par compression à 90 bars pendant 2 min avec une vitesse d'agitation de 100 tr/min.

Le produit de l'Exemple 1 se dissout avec une cinétique de 23 µg.s⁻¹.cm⁻² +/- 11%. A titre de comparaison, la cinétique de dissolution du chlorhydrate correspondant est de 1,6 µg.s⁻¹.cm⁻². Le méthanesulfonate se dissout donc environ 14 fois plus rapidement que le chlorhydrate correspondant.

### Exemple 5 : Compositions pharmaceutiques

### 1000 comprimés dosés à 5 mg du 3-[3-{[4-(4-morpholinylméthyl)-1H-pyrrol-2-yl] méthylène}-2-oxo-2,3-dihydro-1H-indol-5-yl)méthyl]-1,3-thiazolidine-2,4-dione

| | |
|---|---|
| méthanesulfonate, isomère Z (Exemple 1) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. 3-[(3-{[4-(4-morpholinylméthyl)-1*H*-pyrrol-2-yl]méthylène}-2-oxo-2,3-dihydro-1*H*-indol-5-yl)méthyl]-1,3-thiazolidine-2,4-dione méthanesulfonate de formule (II) : 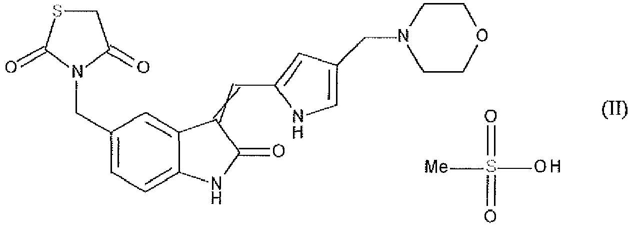 dans laquelle la notation 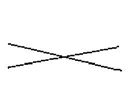 signifie que la double liaison est de configuration Z ou E.

2. Composé selon la revendication 1 qui est l'isomère Z du 3-[(3-{[4-(4-morpholinyl méthyl)-1*H*-pyrrol-2-yl]méthylène}-2-oxo-2,3-dihydro-1*H*-indol-5-yl)méthyl]-1,3-thiazolidine-2,4-dione méthanesulfonate.

3. Composé selon la revendication 1 ou 2 **caractérisé par** son diagramme de diffraction X sur poudre par les angles de Bragg 2 thêta (exprimés en °±0,2) 12,86 ; 15,13 ; 15,50 ; 17,70 ; 18,25 ; 18,71 ; 20,11 ; 21,46 ; 21,67 ; 21,89 ; 22,29 ; 22,58 ; 24,57 ; 25,82 ; 26,33.

4. Composé selon la revendication 1 ou 2 **caractérisé par** les paramètres suivants, obtenus à partir du diagramme de poudre effectué sur le diffractomètre Panalytical Xpert-Pro MPD (anticathode de cuivre) en mode transmission avec un domaine angulaire 3-55° en 2θ, un pas de 0,017° et 35,5 s par pas, qui permet d'identifier les paramètres cristallins suivants:
- paramètres de maille : a = 15.0958(5) Å, b = 18.4586(6) Å, c = 8.8269(2) Å, β = 94.074(1)°, γ = 90°
- groupe d'espace : C 1 c 1 (9)
- volume de la maille : Vₘₐᵢₗₗₑ = 2453.37600 Å³

5. Procédé d'obtention du composé de formule (II) selon la revendication 1 **caractérisé en ce que** l'on utilise comme produit de départ la 3-[(3-{[4-(4-morpholinylméthyl)-1*H-*pyrrol-2-yl]méthylène}-2-oxo-2,3-dihydro-1*H*-indol-5-yl)méthyl]-1,3-thiazolidine-2,4-dione, qui est placée en solution dans un système binaire solvant/eau, dans lequel on ajoute 1 à 2 équivalents molaires d'acide méthane sulfonique que l'on agite jusqu'à précipitation du méthanesulfonate qui est filtré.

6. Procédé d'obtention du composé de formule (II) selon la revendication 1 **caractérisé en ce que** l'on utilise comme produit de départ le 3-[(3-{[4-(4-morpholinylméthyl)-1*H-*pyrrol-2-yl]méthylène}-2-oxo-2,3-dihydro-1*H*-indol-5-yl)méthyl]-1,3-thiazolidine-2,4-dione chlorhydrate, qui est mis en solution dans un système binaire solvant/eau, dont le pH est ramené à 8 par addition d'une base, le sel formé est éliminé par filtration, puis le filtrat chauffé et l'acide méthane sulfonique ajouté, le milieu agité et refroidi jusqu'à précipitation du méthanesulfonate qui est filtré.

7. Composition pharmaceutique contenant le composé de formule (II) selon l'une des revendications 1 à 4 en combinaison avec un ou plusieurs excipients pharmaceutiquement acceptables.

8. Composition pharmaceutique selon la revendication 7 **caractérisée en ce que** le composé de formule (II) est l'isomère Z du 3-[(3-{[4-(4-morpholinyl méthyl)-1*H*-pyrrol-2-yl]méthylène}-2-oxo-2,3-dihydro-1*H*-indol-5-yl)méthyl]-1,3-thiazolidine-2,4-dione méthanesulfonate.

9. Compositions pharmaceutiques selon la revendication 7 ou 8 utiles pour la fabrication de médicaments utiles dans le traitement des cancers du colon, du sein, du foie, des reins, du cerveau, de l'oesophage, les mélanomes, les myélomes, les cancers de l'ovaire, les cancers du poumon non à petites cellules, les cancers du poumon à petites cellules, les cancers de la prostate et du pancréas, les sarcomes.

10. Association d'un composé de formule (II) selon l'une quelconque des revendications 1 à 4 avec un agent anticancéreux choisi parmi les agents génotoxiques, les poisons mitotiques, les anti-métabolites, les inhibiteurs du protéasome, ou les inhibiteurs de kinases.

11. Association selon la revendication 10 **caractérisée en ce que** le composé de formule (II) est l'isomère Z du 3-[(3-{[4-(4-morpholinyl méthyl)-1*H*-pyrrol-2-yl]méthylène}-2-oxo-2,3-dihydro-1*H*-indol-5-yl)méthyl)-1,3-thiazolidine-2,4-dione méthanesulfonate.

12. Utilisation d'une association selon la revendication 10 ou 11 pour la fabrication de médicaments utiles dans le traitement des cancers.

13. Composé de formule (II) selon l'une quelconque des revendications 1 à 4 en association avec une radiothérapie destiné à être utilisé dans le traitement des cancers.

14. Composé de formule (II) pour son utilisation selon la revendication 13 qui est l'isomère Z du 3-[(3-{[4-(4-morpholinylméthyl)-1H-pyrrol-2-yl]méthylène}-2-oxo-2,3-dihydro-1H-indol-5-yl)méthyl]-1,3-thiazolidine-2,4-dione méthanesulfonate.

## Patentansprüche

1. 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion-Methansulfonat der Formel (II): 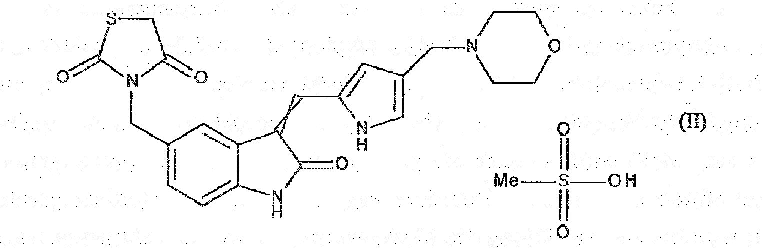 worin das Symbol 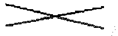 bedeutet, dass die Doppelbindung in der Konfiguration Z oder E vorliegt.

2. Verbindung nach Anspruch 1, nämlich das Z-Isomere von 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion-Methansulfonat.

3. Verbindung nach Anspruch 1 oder 2, **gekennzeichnet durch** ihr Pulverröntgenbeugungsdiagramm mit den Bragg 2 Theta-Winkeln (ausgedrückt als ° ± 0,2) 12,86; 15,13; 15,50; 17,70; 18,25; 18,71; 20,11; 21,46; 21,67; 21,89; 22,29; 22,58; 24,57; 25,82; 26,33.

4. Verbindung nach Anspruch 1 oder 2, **gekennzeichnet durch** die folgenden Parameter: erhalten ausgehend von dem auf dem Diffraktometer Panalytical Xpert-Pro MPD (Kupfer-Antikathode) im Transmissionsmodus mit einem Winkelbereich von 3-55° 2θ, einer Schrittweite von 0,017° und 35,5 s pro Schritt, welche die Identifizierung der folgenden Kristallparameter ermöglichen:
- Gitterparameter: a = 15,0958(5) Å, b = 18,4586(6) Å, c = 8,8269(2) Å, β = 94,074(1)°, γ = 90°
- Raumgruppe: C 1 c 1 (9)
- Zellvolumen: V_{Zelle} = 2453,37600 Å³.

5. Verfahren zur Herstellung der Verbindung der Formel (II) nach Anspruch 1, **dadurch gekennzeichnet, dass** man als Ausgangsprodukt 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion verwendet, welches in einem binären Lösungsmittel/Wasser-System gelöst wird, zu dem man 1 bis 2 Mol-Äquivalente Methansulfonsäure zusetzt und rührt bis zur Ausfällung des Methansulfonats, welches abfiltriert wird.

6. Verfahren zur Herstellung der Verbindung der Formel (II) nach Anspruch 1, **dadurch gekennzeichnet, dass** man als Ausgangsprodukt 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion-Hydrochlorid verwendet, welches in einem binären Lösungsmittel/Wasser-System gelöst wird, dessen pH-Wert durch Zugabe einer Base auf 8 eingestellt wird, wonach das gebildete Salz durch Filtration abgetrennt wird, das Filtrat erhitzt und Methansulfonsäure zugesetzt wird, das Medium gerührt und abgekühlt wird bis zur Ausfällung des Methansulfonats, welches abfiltriert wird.

7. Pharmazeutische Zubereitung enthaltend die Verbindung der Formel (II) nach einem der Ansprüche 1 bis 4 in Kombination mit einem oder mehreren pharmazeutisch annehmbaren Hilfsstoffen.

8. Pharmazeutische Zubereitung nach Anspruch 7, **dadurch gekennzeichnet, dass** die Verbindung der Formel (II) das Z-Isomere von 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion-Methansulfonat ist.

9. Pharmazeutische Zubereitungen nach Anspruch 7 oder 8, nützlich zur Herstellung von Arzneimitteln, die nützlich sind bei der Behandlung von Darmkrebs, Brustkrebst, Leberkrebs, Nierenkrebs, Gehirnkrebs, Speiseröhrenkrebs, Melanomen, Myelomen, Ovarialkrebsen, nicht kleinzelligen Lungenkrebsen, kleinzelligen Lungenkrebsen, Prostata- und Pankreaskrebsen, Sarkomen.

10. Kombination einer Verbindung der Formel (II) nach einem der Ansprüche 1 bis 4, mit einem Antikrebsmittel ausgewählt aus genotoxischen Mitteln, Mitosegiften, Anti-Metaboliten, Proteasom-Inhibitoren oder Kinase-Inhibitoren.

11. Kombination nach Anspruch 10, **dadurch gekennzeichnet, dass** die Verbindung der Formel (II) das Z-Isomere von 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion-Methansulfonat ist.

12. Verwendung einer Kombination nach Anspruch 10 oder 11 für die Herstellung von Arzneimitteln, die nützlich sind bei der Behandlung von Krebserkrankungen.

13. Verbindung der Formel (II) nach einem der Ansprüche 1 bis 4 in Kombination mit einer Strahlentherapie zur Verwendung bei der Behandlung von Krebserkrankungen.

14. Verbindung der Formel (II) zur Verwendung nach Anspruch 13, welche das Z-Isomere von 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion-Methansulfonat ist.

## Claims

1. 3-[(3-{[4-(4-Morpholinylmethyl)-1*H*-pyrrol-2-yl]methylene}-2-oxo-2,3-dihydro-1*H*-indol-5-yl)methyl]-1,3-thiazolidine-2,4-dione methanesulphonate of formula (II): 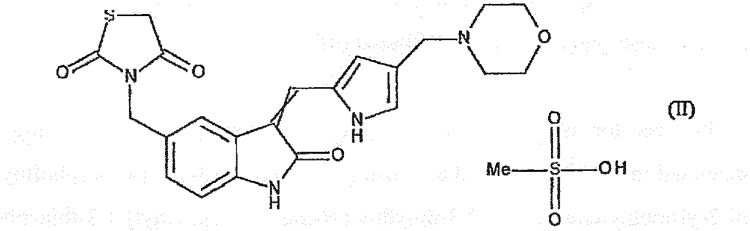 wherein the notation 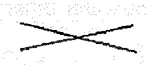 means that the double bond has the Z or E configuration.

2. Compound according to claim 1 which is the Z isomer of 3-[(3-{[4-(4-morpholinyl-methyl)-1*H-*pyrrol-2-yl]methylene}-2-oxo-2,3-dihydro-1*H-*indol-5-yl)methyl]-1,3-thiazolidine-2,4-dione methanesulphonate.

3. Compound according to claim 1 or 2, **characterised by** its X-ray powder diffraction diagram by the Bragg's angles 2 theta (expressed in °±0.2) 12.86; 15.13; 15.50; 17.70; 18.25; 18.71; 20.11; 21.46; 21.67; 21.89; 22.29; 22.58; 24.57; 25.82; 26.33.

4. Compound according to claim 1 or 2, **characterised by** the following parameters, obtained from the powder diagram performed on a Panalytical Xpert-Pro MPD diffractometer (copper anticathode) in transmission mode with an angle range 3-55° 2θ, a step of 0.017° and 35.5 s per step, which allows the following crystal parameters to be identified:
- unit cell parameters: a = 15.0958(5) A, b = 18.4586(6) A, c = 8.8269(2) Å, β = 94.074(1)°, γ = 90°
- space group: C 1 c 1 (9)
- volume of the unit cell: V_{unit cell} = 2453.37600 Å³.

5. Process for obtaining the compound of formula (II) according to claim 1, **characterised in that** there is used as starting material 3-[(3-{[4-(4-morpholinylmethyl)-1*H-*pyrrol-2-yl]methylene}-2-oxo-2,3-dihydro-1*H*-indol-5-yl)methyl]-1,3-thiazolidine-2,4-dione, which is placed in solution in a solvent/water binary system, to which there is added from 1 to 2 molar equivalents of methanesulphonic acid, which is stirred until precipitation of the methanesulphonate, which is filtered off.

6. Process for obtaining the compound of formula (II) according to claim 1, **characterised in that** there is used as starting material 3-[(3-{[4-(4-morpholinylmethyl)-1*H-*pyrrol-2-yl]methylene}-2-oxo-2,3-dihydro-1*H-*indol-5-yl)methyl]-1,3-thiazolidine-2,4-dione hydrochloride, which is dissolved in a solvent/water binary system, the pH of which is brought to 8 by addition of a base, the salt that forms is removed by filtration, and then the filtrate is heated and methanesulphonic acid is added, the mixture is stirred and cooled until precipitation of the methanesulphonate, which is filtered off.

7. Pharmaceutical composition comprising the compound of formula (II) according to any one of claims 1 to 4 in combination with one or more pharmaceutically acceptable excipients.

8. Pharmaceutical composition according to claim 7, **characterised in that** the compound of formula (II) is the Z isomer of 3-[(3-{[4-(4-morpholinylmethyl)-1*H*-pyrrol-2-yl]methylene}-2-oxo-2,3-dihydro-1*H-*indol-5-yl)methyl]-1,3-thiazolidine-2,4-dione methanesulphonate.

9. Pharmaceutical compositions according to claim 7 or 8 for use for the manufacture of medicaments for use in the treatment of cancers of the colon, breast, liver, kidneys, brain and oesophagus, melanomas, myelomas, ovarian cancers, non-small-cell lung cancers, small-cell lung cancers, prostate and pancreatic cancers, sarcomas.

10. Association of a compound of formula (II) according to any one of claims 1 to 4 with an anti-cancer agent chosen from genotoxic agents, mitotic poisons, anti-metabolites, proteasome inhibitors, and kinase inhibitors.

11. Association according to claim 10, **characterised in that** the compound of formula (II) is the Z isomer of 3-[(3-{[4-(4-morpholinylmethyl)-1*H-*pyrrol-2-yl]methylene}-2-oxo-2,3-dihydro-1*H-*indol-5-yl)methyl]-1,3-thiazolidine-2,4-dione methanesulphonate.

12. Use of an association according to claim 10 or 11 for the manufacture of medicaments for use in the treatment of cancers.

13. Compound of formula (II) according to any one of claims 1 to 4 in association with radiotherapy, for use in the treatment of cancers.

14. Compound of formula (II) for its use according to claim 13 which is the Z isomer of 3-[(3-{[4-(4-morpholinylmethyl)-1*H-*pyrrol-2-yl]methylene}-2-oxo-2,3-dihydro-1*H-*indol-5-yl)methyl]-1,3-thiazolidine-2,4-dione methanesulphonate.
